# FASCICULE DE BREVET EUROPEEN

(11) **EP 3 129 084 B1**
(45) Date de publication et mention de la délivrance du brevet: **28.03.2018**
(21) Numéro de dépôt: 15723256.2
(22) Date de dépôt: 09.04.2015
(51) Int. Cl.: A61M 5/20, A61M 5/32

(54) **AUTOINJECTEUR**
AUTOINJEKTOR
AUTOINJECTOR

(30) Priorité: 11.04.2014 FR 1453251
(43) Date de publication de la demande: 15.02.2017
(73) Titulaire: APTAR France SAS, 27110 Le Neubourg (FR)
(72) Inventeur: SAUSSAYE, Anthony, 27400 Louviers (FR)
(74) Mandataire: CAPRI
(86) Numéro de dépôt international: PCT/FR2015/050940
(87) Numéro de publication internationale: WO 2015/155484

(56) Documents cités:
- WO-A1-2010/108116
- WO-A1-2013/175137
- WO-A1-2013/175144
- WO-A1-2013/175148

## Description

La présente invention concerne un autoinjecteur.

Les autoinjecteurs sont bien connus dans l'état de la technique. Ces dispositifs ont principalement pour but de réaliser une injection automatique du contenu d'une seringue à l'intérieur du corps d'un patient. Divers systèmes existent pour rendre automatique l'injection du produit fluide contenu dans la seringue. Les autoinjecteurs sont des dispositifs relativement complexes qui doivent répondre à un certain nombre d'exigences de contraintes pour être fiables. La robustesse du dispositif, sa maniabilité, sa fiabilité et sa facilité d'utilisation pour l'utilisateur sont également des éléments importants. Par ailleurs, la plupart de ces autoinjecteurs étant à usage unique, le coût de fabrication et d'assemblage est également un facteur dont il faut tenir compte.

Il existe de nombreux autoinjecteurs sur le marché, qui présentent toutefois tous un certain nombre d'inconvénients.

Ainsi, pour éviter un déclenchement intempestif de l'autoinjecteur, par exemple pendant un transport ou pendant le stockage, les dispositifs doivent comporter des moyens de verrouillage fiables. De même, lorsqu'un utilisateur souhaite utiliser l'autoinjecteur et qu'il déverrouille le dispositif, par exemple en retirant le capot, le dispositif ne doit pas se déclencher de manière intempestive mais seulement au moment où l'utilisateur le souhaite réellement, c'est-à-dire au moment où il l'applique contre la partie du corps dans laquelle il veut réaliser l'injection. Or, notamment quand les personnes utilisant l'autoinjecteur sont des personnes âgées ou handicapées, il peut arriver que l'utilisateur laisse tomber le dispositif au moment où il souhaite l'utiliser. Il est souhaitable que dans un tel cas l'autoinjecteur ne se déclenche pas tout seul. Il est donc important de prévoir une serrure de déclenchement fiable. D'un autre côté, il ne faut pas que l'utilisation de l'autoinjecteur devienne trop difficile, ce qui empêcherait des personnes faibles de l'utiliser. Il est donc difficile de trouver le bon compromis entre la sécurité du verrouillage et la facilité d'utilisation et d'actionnement de l'autoinjecteur. C'est un des objectifs de la présente invention de répondre à ce problème.

Les documents WO 2013/175148, WO 2013/175144 et WO 2010/108116 décrivent des dispositifs de l'état de la technique.

La présente invention a pour but de fournir un autoinjecteur qui ne reproduit pas les inconvénients susmentionnés, et qui permet de répondre aux différentes exigences et contraintes importantes pour une utilisation sûre et fiable de l'autoinjecteur.

La présente invention a également pour but de fournir un autoinjecteur qui comporte une serrure d'injection fiable, résistante aux actionnements non souhaités, et aisément déclenchable sans efforts excessifs.

La présente invention a aussi pour but de fournir un autoinjecteur qui soit simple et peu coûteux à fabriquer et à assembler.

La présente invention a donc pour objet un autoinjecteur comportant un corps recevant un réservoir, ledit réservoir contenant du produit fluide et comportant un piston et une aiguille, tel qu'une seringue pré-remplie, ledit autoinjecteur comportant une tige de piston adaptée à coopérer avec le piston dudit réservoir, ladite tige de piston étant déplaçable entre une position chargée et une position d'injection dans laquelle ladite tige de piston a déplacé le piston du réservoir pour injecter le produit fluide à travers l'aiguille, un ressort d'injection étant prévu pour solliciter ladite tige de piston vers sa position d'injection, l'autoinjecteur comportant une serrure d'injection qui bloque ladite tige de piston dans sa position chargée, ladite serrure d'injection comportant un manchon de commande, ledit manchon de commande contenant ladite tige de piston et ledit ressort d'injection, ladite tige de piston comportant un évidement radial recevant au moins un élément de blocage sensiblement sphérique, tel qu'une bille, mobile entre une position de blocage et une position de déblocage, ledit au moins un élément de blocage étant sollicité radialement vers l'extérieur par ladite tige de piston et étant retenu en position de blocage par une bague de blocage, ladite bague de blocage étant déplaçable par rapport à ladite tige de piston pour libérer ledit au moins un élément de blocage et ainsi débloquer ladite serrure d'injection, permettant audit ressort d'injection de déplacer ladite tige de piston vers sa position d'injection, un organe support étant fixé, notamment encliqueté, dans ledit manchon de commande, ledit organe support comportant un anneau dont un côté axial est en contact avec ledit ressort d'injection et l'autre côté axial supporte ledit au moins un élément de blocage sensiblement sphérique, ladite bague de blocage étant engagée, notamment encliquetée, sur ledit organe support, ladite bague de blocage étant désengagée dudit organe support par ledit manchon de commande lorsque ledit manchon de commande est déplacé axialement lors de l'actionnement de l'autoinjecteur, pour débloquer ladite serrure d'injection.

Avantageusement, ladite bague de blocage comporte des ergots, de préférence de forme arrondie, qui sont, en position de blocage de la serrure d'injection, encliquetés sous une rampe inclinée dudit organe support.

Avantageusement, ladite bague de blocage comporte un bord inférieur chanfreiné pour solliciter ledit au moins un élément de blocage radialement vers l'intérieur lors de l'assemblage de ladite bague de blocage sur ledit organe support.

Avantageusement, ledit organe support comporte des premières projections radiales coopérant avec un épaulement dudit manchon de commande pour fixer ledit organe support sur ledit manchon de commande.

Avantageusement, un élément indicateur est fixé, notamment encliqueté, sur ledit organe support, ledit élément indicateur coopérant après injection avec le corps pour fournir à l'utilisateur une indication sonore et/ou tactile et/ou visuelle.

Avantageusement, ledit organe support comporte des secondes projections radiales coopérant avec des fenêtres dudit élément indicateur pour fixer ledit élément indicateur sur ledit organe support.

Avantageusement, ledit autoinjecteur comporte un manchon actionneur comportant une extrémité de contact destinée à venir en contact avec le corps de l'utilisateur, ledit manchon actionneur s'étendant au moins partiellement à l'intérieur dudit corps et étant déplaçable par rapport audit corps entre des positions projetées, dans lesquelles ledit manchon actionneur fait au moins partiellement saillie hors dudit corps, et une position d'actionnement, dans laquelle ledit manchon actionneur est axialement déplacé vers l'intérieur dudit corps, ledit manchon actionneur étant dans une première position projetée avant actionnement de l'autoinjecteur et dans une seconde position projetée après actionnement de l'autoinjecteur.

Avantageusement, ledit manchon actionneur comporte un bord supérieur qui vient en contact dudit manchon de commande lors de l'actionnement, de sorte que ledit manchon de commande est déplacé axialement par ledit manchon actionneur pour débloquer ladite serrure d'injection.

Ces caractéristiques et avantages et d'autres de la présente invention apparaîtront plus clairement au cours de la description détaillée suivante, faite en référence aux dessins joints, donnés à titre d'exemples non limitatifs, et sur lesquels :
Les figures 1a et 1b sont des vues en section transversale selon deux plans de coupe différents d'un autoinjecteur selon un mode de réalisation avantageux de la présente invention, avant utilisation,
Les figures 2a et b sont des vues similaires respectivement à celles des figures 1a et 1b, après retrait du capot de protection et avant actionnement,
Les figures 3a et 3b sont des vues similaires respectivement à celles des figures 1 a et 1b, en début d'actionnement,
Les figures 4a et 4b sont des vues similaires respectivement à celles des figures 1a et 1b, en cours d'actionnement et en début d'injection,
Les figures 5a et 5b sont des vues similaires respectivement à celles des figures 1a et 1b, après injection,
Les figures 6a et 6b sont des vues similaires respectivement à celles des figures 1a et 1b, en fin d'actionnement,
Les figures 7a, 7b et 7c sont des vues de détail en section transversale selon trois plans de coupe différents de l'autoinjecteur des figures 1 à 6, avant actionnement, les figures 7a et 7b étant des vues de détail en section transversale selon les mêmes plans de coupe que les figures 1 à 6, et la figure 7c étant une vue de détail supplémentaire selon un autre plan de coupe non représenté sur les figures 1 à 6,
Les figures 8a, 8b et 8c sont des vues similaires respectivement à celles des figures 7a, 7b et 7c, en début d'actionnement,
Les figures 9a, 9b et 9c sont des vues similaires respectivement à celles des figures 7a, 7b et 7c, avant déclenchement de la serrure d'injection,
Les figures 10a, 10b et 10c sont des vues similaires respectivement à celles des figures 7a, 7b et 7c, en cours de déclenchement de la serrure d'injection mais avant injection,
Les figures 11a, 11b et 11c sont des vues similaires respectivement à celles des figures 7a, 7b et 7c, après déclenchement de la serrure d'injection en début d'injection,
Les figures 12a, 12b et 12c sont des vues similaires respectivement à celles des figures 7a, 7b et 7c, en fin d'injection mais avant déclenchement de l'indicateur de fin d'injection,
Les figures 13a, 13b et 13c sont des vues similaires respectivement à celles des figures 7a, 7b et 7c, en fin d'injection mais après déclenchement de l'indicateur de fin d'injection,
Les figures 14a, 14b et 14c sont des vues similaires respectivement à celles des figures 7a, 7b et 7c, après injection,
Les figures 15a à 15g sont des vues schématiques de détail des séquences d'assemblage de la serrure d'injection,
La figure 16 est une vue schématique en perspective du manchon de commande de l'autoinjecteur des figures 1 à 14,
La figure 17 est une vue schématique en perspective de la tige de piston de l'autoinjecteur des figures 1 à 14,
Les figures 18a et 18b sont des vues schématiques en perspective selon deux orientations différentes de la bague de blocage de l'autoinjecteur des figures 1 à 14,
Les figures 19a et 19b sont des vues schématiques en perspective selon deux orientations différentes de l'organe support de l'autoinjecteur des figures 1 à 14,
La figure 20 est une vue schématique en perspective de l'élément indicateur de l'autoinjecteur des figures 1 à 14,
Les figures 21a et 21b illustrent schématiquement l'assemblage de l'organe support dans le manchon de commande, dans le mode de réalisation des figures 1 à 14,
Les figures 22a à 22c illustrent schématiquement l'assemblage des éléments de blocage de la serrure d'injection, dans le mode de réalisation des figures 1 à 14, et
Les figures 23a à 23c illustrent schématiquement l'assemblage de la bague de blocage sur l'organe support, dans le mode de réalisation des figures 1 à 14.

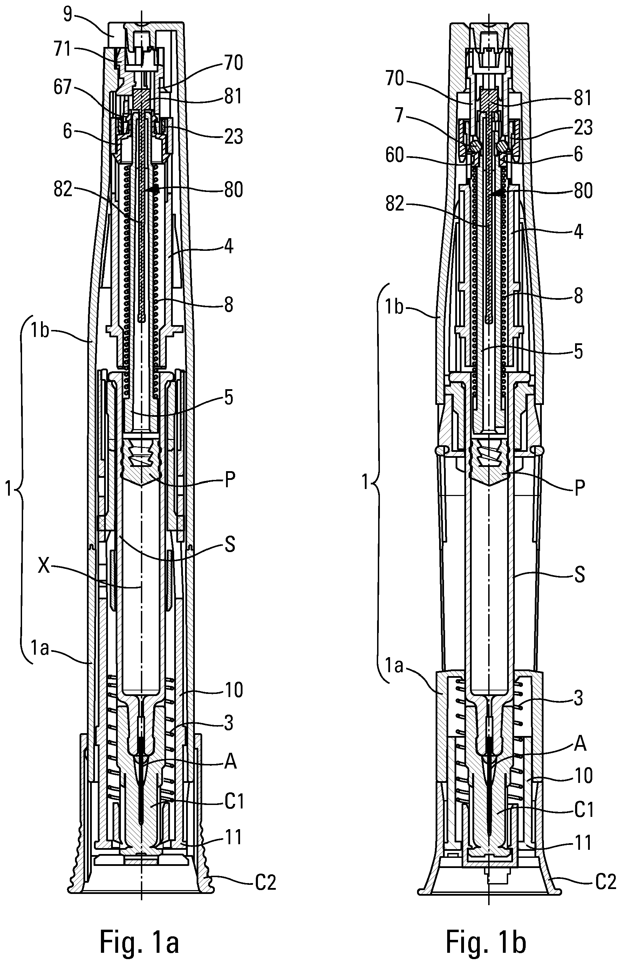

Dans la description ci-après, les termes "supérieur", "inférieur", "haut" et "bas" se réfèrent aux positions représentées sur les figures 1a à 15g et 21a à 23c. Les termes "axial" et "radial" se réfèrent à l'axe central longitudinal X, représenté notamment sur la figure 1a.

L'autoinjecteur va être décrit ci-après en référence à un mode de réalisation avantageux de celui-ci. Il est toutefois à noter que les autoinjecteurs, qui sont des appareils complexes, comprennent plusieurs modules pour réaliser plusieurs fonctions. Ces divers modules peuvent être utilisés séparément et indépendamment les uns des autres, sans être nécessairement combinés aux autres modules, et pourraient notamment être utilisés dans des autoinjecteurs de forme différente de celle représentée sur les dessins. De plus, il est à noter que les dessins sont des vues schématiques qui ne représentent pas forcément la forme exacte des composants d'un autoinjecteur, et ne sont pas forcément à l'échelle, notamment dans des buts de clarté. Par ailleurs, les dessins ne représentent pas forcément tous les éléments constitutifs d'un autoinjecteur, mais seulement les éléments nécessaires au fonctionnement de la présente invention. Ainsi, divers éléments et modules supplémentaires et/ou complémentaires pourraient être associés à l'autoinjecteur représenté sur les figures.

L'autoinjecteur représenté sur les figures comporte un corps 1 dans lequel coulisse axialement un manchon actionneur 10, dont l'extrémité inférieure 11 est destinée à venir en contact avec le corps du patient autour de la zone d'injection. Dans l'exemple représenté sur les figures, l'autoinjecteur comporte un corps inférieur 1a et un corps supérieur 1b qui sont assemblés pour former le corps 1 de l'autoinjecteur, comme indiqué sur les figures 1a et 1b. Ci-après, et dans les autres figures, le terme "corps" et la référence numérique "1" seront utilisés pour désigner ledit corps unitaire formé par l'assemblage dudit corps inférieur 1a avec ledit corps supérieur 1b.

Un réservoir S peut être inséré dans ledit autoinjecteur. Ce réservoir S contient du produit fluide, et comporte un piston P et une aiguille A. Le piston P est adapté à se déplacer dans ledit réservoir S pour injecter le produit fluide à travers ladite aiguille A.

La présente description sera faite en référence à une seringue S, qui peut être de tout type. Plus généralement, il est entendu que le terme « seringue » dans la présente description englobe tout type de réservoir associé à une aiguille. De préférence, le réservoir S est une seringue pré-remplie.

Avant actionnement de l'autoinjecteur, l'aiguille A de la seringue S est de préférence protégée par un capuchon C1, l'autoinjecteur comportant un capot C2 que l'utilisateur peut retirer avant actionnement. Le retrait du capot C2 provoque le retrait du capuchon C1.

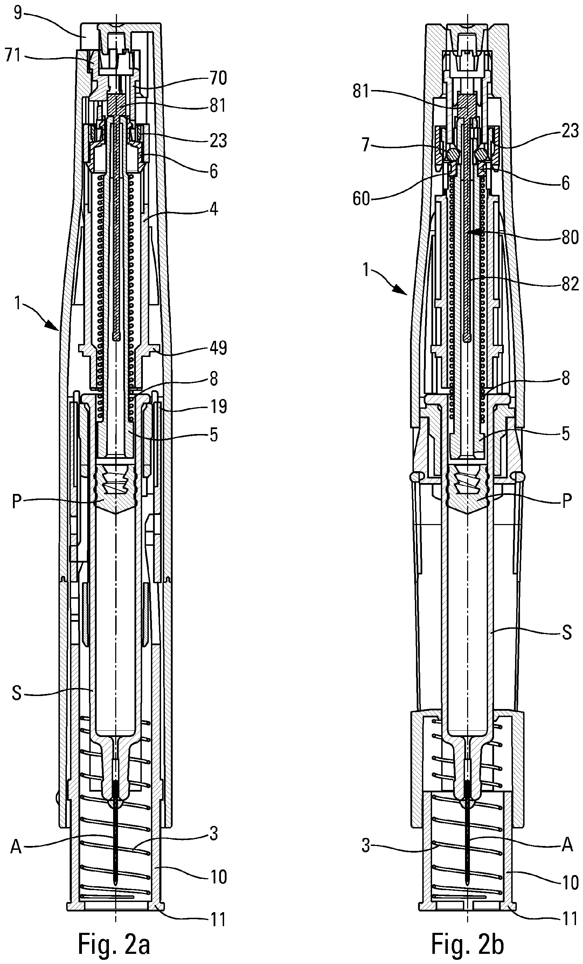

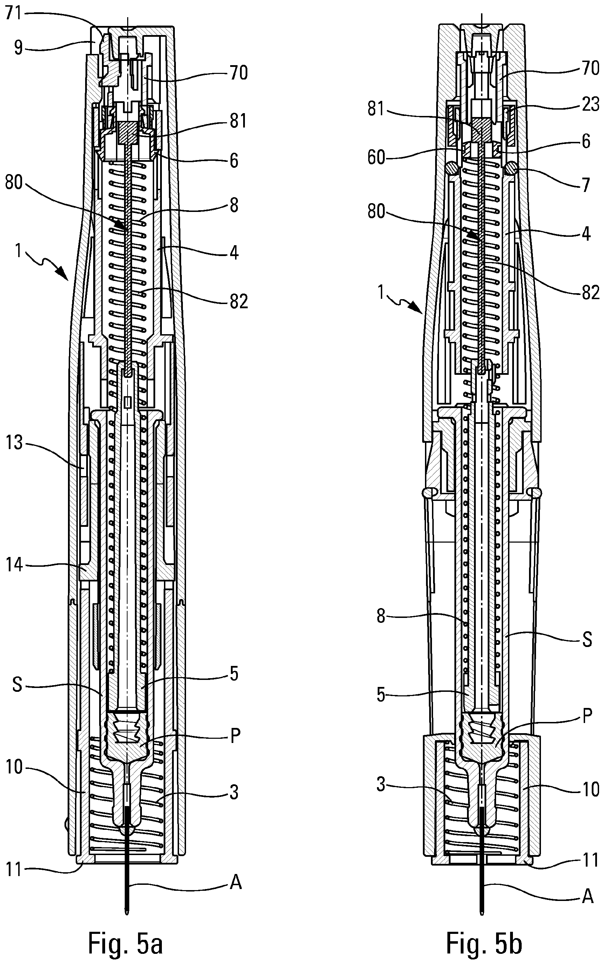

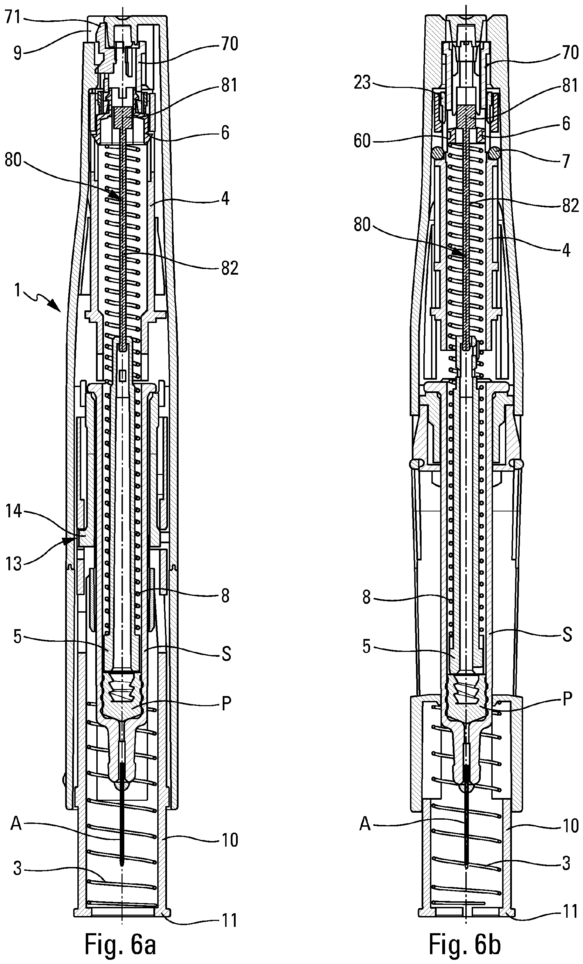

Avant actionnement, le manchon actionneur 10 est dans une première position projetée dans laquelle il entoure l'aiguille A, comme représenté sur les figures 2a et 2b. Lors de l'actionnement, le manchon actionneur 10 coulisse à l'intérieur du corps 1 vers une position d'actionnement, pour exposer l'aiguille A et permettre le piquage puis l'injection du produit fluide. Après l'injection, le manchon actionneur 10 revient dans une seconde position projetée, dans laquelle il est à nouveau disposé autour de l'aiguille A, pour éviter tout risque de blessure avec ladite aiguille, comme représenté sur les figures 6a et 6b. Le manchon actionneur 10 est avantageusement sollicité vers ses positions projetées par un ressort 3, qui peut être de type quelconque. Avantageusement, dans ladite seconde position projetée après injection, ledit manchon actionneur 10 est verrouillé, et ne peut plus être déplacé axialement vers l'intérieur dudit corps 1. Ce verrouillage peut par exemple être réalisé par des pattes 14, solidaires du corps 1 ou du réservoir S, et coopérant avec des ouvertures 13 dudit manchon actionneur 10 lorsque celui-ci atteint sa seconde position projetée. Dans l'exemple représenté, les pattes 14 sont solidaires du réservoir S, comme visible notamment sur les figures 5a et 6a. Ce verrouillage, qui n'est pas essentiel au fonctionnement de la présente invention, ne sera pas décrit plus en détail ci-après. Il pourrait être réalisé différemment du mode de réalisation particulier représenté sur les dessins. En particulier, il pourrait être réalisé conformément aux enseignements des documents WO 2013/175140 ou WO 2013/175142.

L'autoinjecteur comporte également un système d'injection automatique, comportant notamment une tige de piston 5 adaptée à coopérer avec le piston P pour le déplacer dans le réservoir S pour distribuer le produit fluide à travers l'aiguille A. Cette tige de piston 5 est classiquement sollicitée par un ressort d'injection 8 vers sa position de distribution, et retenue avant actionnement dans sa position de repos par une serrure d'injection appropriée.

Les figures 1a à 14c illustrent schématiquement une serrure d'injection avantageuse selon l'invention, comprenant au moins un élément de blocage 7 retenu en position de blocage par une bague de blocage 23. Le déclenchement de ladite serrure d'injection provoque l'actionnement desdits moyens d'injection et donc l'injection du produit fluide à travers l'aiguille.

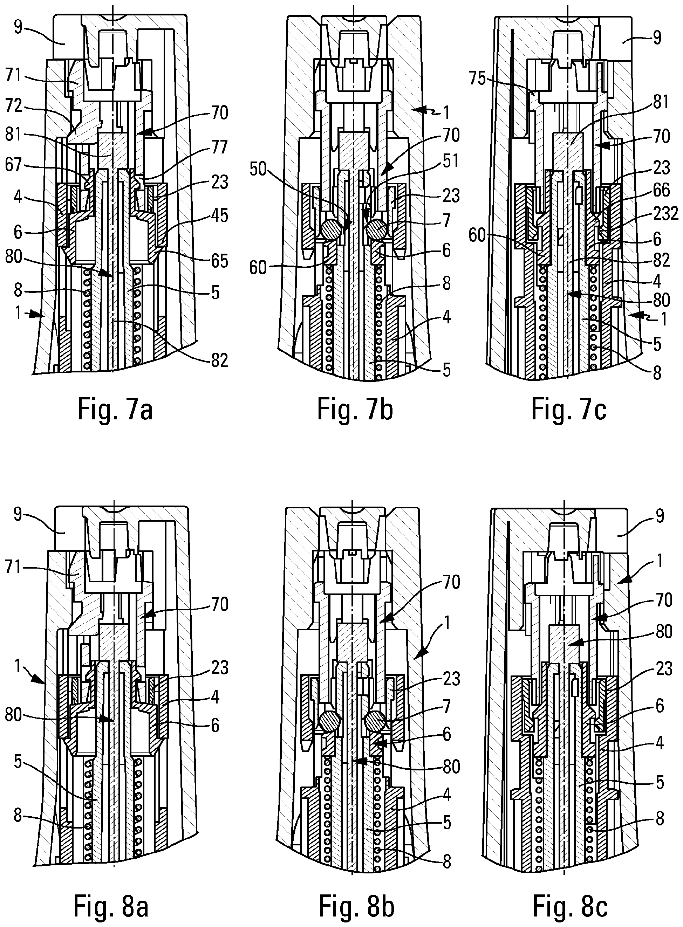

Comme représenté notamment sur la figure 7b, ladite serrure d'injection comporte un manchon de commande 4 disposé dans ledit corps 1, ledit manchon de commande 4 contenant ladite tige de piston 5 et ledit ressort d'injection 8, ladite tige de piston 5 comportant un évidement radial 50 recevant au moins un élément de blocage 7 mobile entre une position de blocage et une position de déblocage. Ledit au moins un élément de blocage 7 est de préférence de forme sensiblement sphérique, tel qu'une bille. Dans la variante représentée, il y a deux éléments de blocage 7 sous forme de billes, mais un nombre différent d'éléments de blocage est envisageable. Ci-après, il sera fait référence à des billes en tant qu'éléments de blocage 7. Lesdites billes 7 sont sollicitées radialement vers l'extérieur par ladite tige de piston 5 et sont retenues en position de blocage par la bague de blocage 23. Cette bague de blocage 23 est déplaçable axialement par rapport à ladite tige de piston 5 entre une position de verrouillage, dans laquelle elle maintient lesdites billes 7 en position de blocage, et une position de déverrouillage, dans laquelle lesdites billes 7 sont libérés pour ainsi débloquer ladite serrure d'injection, permettant audit ressort d'injection 8 de déplacer ladite tige de piston 5 vers sa position d'injection.

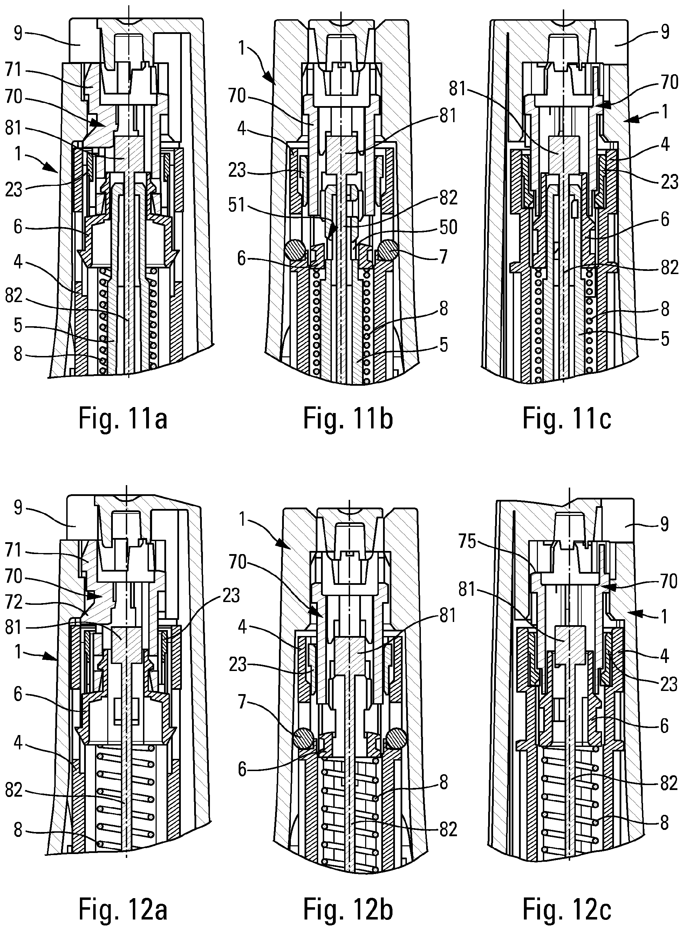

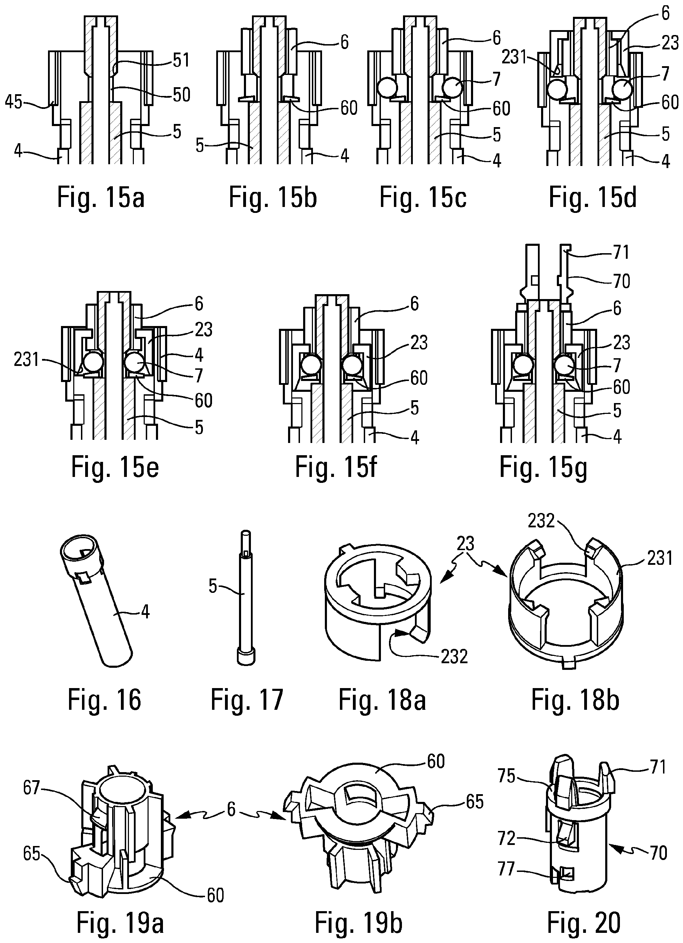

Les figures 7a à 9c montrent la serrure d'injection dans la position de blocage. Le ressort d'injection 8 coopère d'une part avec la tige de piston 5 et d'autre part avec un organe support 6. Cet organe support 6 comporte un anneau 60 disposé autour de ladite tige de piston 5. La tige de piston 5 comporte un évidement périphérique 50, pourvu avantageusement d'une surface inclinée 51, formé par un rétrécissement du diamètre de ladite tige de piston 5. Ceci est mieux visible sur les figures 11b et 15a. Cette tige de piston 5 est disposée à l'intérieur du manchon de commande 4 et est susceptible d'être déplacée axialement vers le bas, dans la position représentée sur les figures 1a à 15g, pour déplacer le piston P à l'intérieur de la seringue S et ainsi distribuer le produit fluide contenu dans ladite seringue S à travers l'aiguille A.

Comme visible notamment sur la figure 7b et les figures 15f et 15g, les billes 7, en position de blocage de la serrure d'injection, sont disposées dans ledit évidement 50 formé dans la tige de piston 5 et coopère donc avec d'une part la paroi inclinée 51 de la tige de piston 5, d'autre part la surface supérieure dudit anneau 60 dudit organe support 6 et avec ladite bague de blocage 23.

La surface inclinée 51 de la tige de piston est au contact des billes 7 de sorte que sous l'effet du ressort comprimé 8, ladite surface inclinée 51 exerce une force de réaction sur les billes 7, cette force n'étant pas exactement axiale mais dirigée légèrement vers l'extérieur, sollicitant ainsi les billes 7 radialement vers l'extérieur de la position de blocage de la figure 20.

La bague de blocage 23 est prévue radialement à l'extérieur des billes 7 pour bloquer radialement lesdites billes dans la position de blocage.

L'organe support 6 transmet la force du ressort 8 aux billes 7, et la bague de blocage 23 exerce une force de réaction sur les billes 7 pour empêcher un déplacement radial de celles-ci. Ainsi, ce sont les billes 7 qui supportent tous les efforts exercés sur la serrure dans la position de blocage, avec un équilibre en trois points sous l'effet des trois forces susmentionnées. Une telle serrure est particulièrement stable et robuste, et permet notamment de résister aux "drops tests". Ces tests simulent le fait de laisser tomber l'autoinjecteur par terre après avoir retiré le capot C2, l'objectif étant d'éviter un déclenchement de la serrure d'injection lors de cette chute. En particulier, aucune force n'est exercée sur les pièces de structure de l'autoinjecteur, tel que le corps 1 ou le manchon actionneur 10. Cette serrure permet ainsi d'éviter un risque de démontage et/ou d'actionnement intempestif de l'autoinjecteur pendant un transport ou une manipulation.

Lorsque l'aiguille A de la seringue S a pénétré le corps de l'utilisateur, la bague de blocage 23 est déplacée axialement vers le haut, comme cela sera plus amplement décrit ci-après. Ceci a pour effet de libérer les billes 7 de leur position de blocage, celles-ci étant alors déplacées radialement vers l'extérieur. La tige de piston 5 n'est alors plus retenue par les billes 7 et elle est donc déplacée axialement vers le bas pour réaliser l'injection du produit. Après déclenchement de la serrure d'injection, les billes 7 ne peuvent plus revenir radialement vers l'intérieur, empêchées par le manchon de commande 4, comme visible sur les figure 11b, 12b, 13b et 14b.

La serrure d'injection représentée sur les figures permet de déverrouiller une force importante exercée par un ressort comprimé, en l'occurrence le ressort d'injection 8, en exerçant une force relativement faible et aisément maitrisable sur la bague de blocage 23. En particulier, la force nécessaire pour déplacer ladite bague de blocage 23 en position de déblocage peut représenter seulement 30-50%, voire moins, de la force exercée par le ressort d'injection 8. Ceci représente un rendement important qui garantit un actionnement aisé et fiable du dispositif, ne nécessitant pas d'efforts excessifs de l'utilisateur.

Avantageusement, l'autoinjecteur comporte un dispositif d'indication visuel, sonore et/ou tactile pour indiquer par un son audible, par une vibration ou par une indication visuelle et/ou tactile à l'utilisateur que la phase d'injection est terminée.

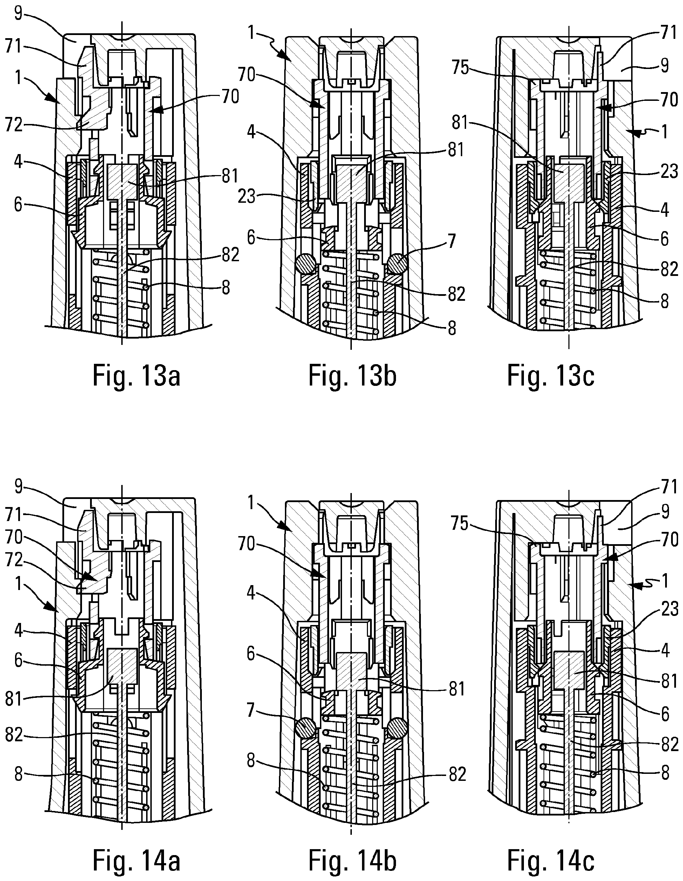

Dans le mode de réalisation représenté, le dispositif d'indication comporte un élément indicateur 70 qui est solidaire de l'organe support 6 sur lequel s'appuie le ressort d'injection 8. La figure 20 montre cet élément indicateur, qui comporte une ou plusieurs pattes 71, en l'occurrence trois pattes. Le fonctionnement est similaire au dispositif décrit dans le document WO 2014/049214, avec une clé 80 comportant une tête 81 et une tige 82, ladite tête bloquant la déformation radiale d'une patte déformable 72 formée sur l'élément indicateur 70, ce qui bloque l'élément indicateur 70 par rapport au corps 1. Dans la variante représentée, il y a une unique patte déformable 72, mais un nombre différent de pattes déformables est envisageable. En fin d'injection, la tige de piston 5 fait coulisser ladite clé 80 axialement vers le bas, par traction ladite tige 82, de sorte que ladite tête 81 de la clé 80 va libérer ladite patte déformable 72, qui, en se déformant radialement vers l'intérieur, va permettre à une partie de bord 75 dudit élément indicateur 70 d'être projetée contre ledit corps 1, sous l'effet du ressort d'injection 8, créant une indication sonore et/ou tactile, notamment en faisant vibrer l'autoinjecteur. Les figures 13c et 14c illustrent notamment ce contact entre la partie de bord 75 de l'élément indicateur 70 et le corps 1. Avantageusement, une indication visuelle est aussi fournie par une ou plusieurs fenêtres de visualisation 9, en l'occurrence trois, du corps 1 qui montrent les pattes 71 de l'élément indicateur 70.

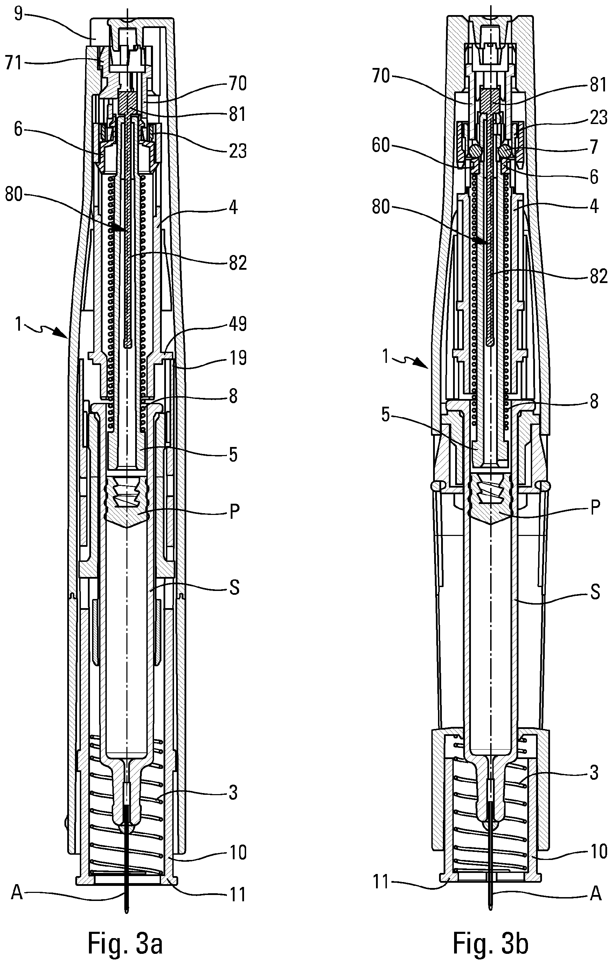

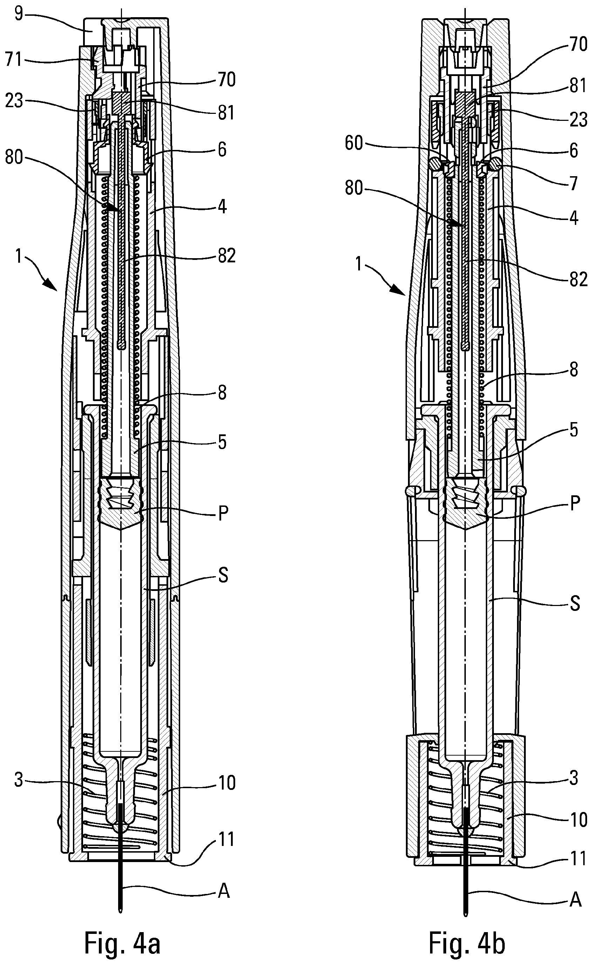

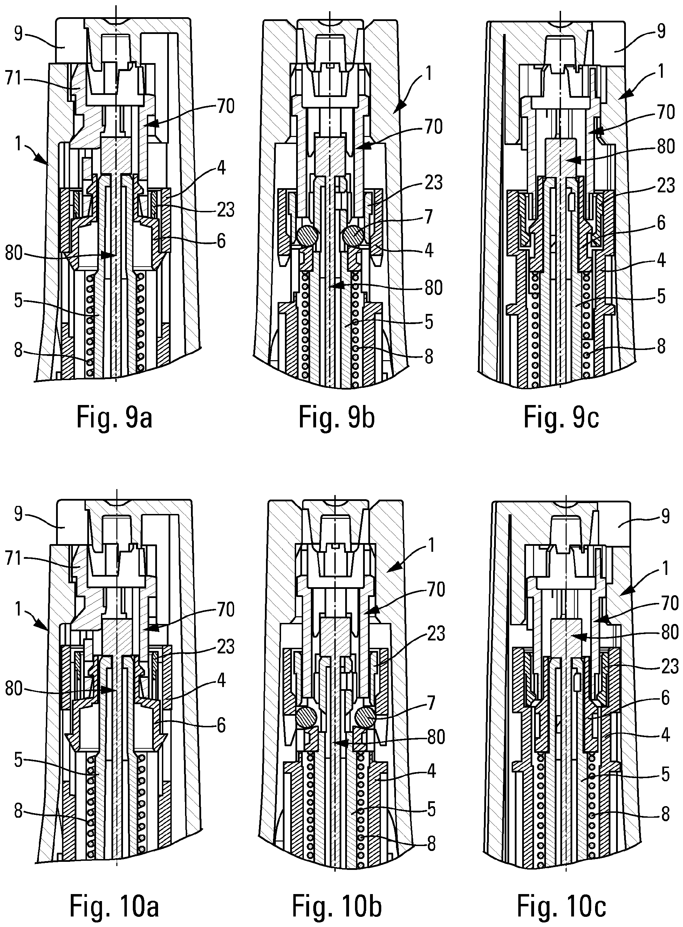

Le fonctionnement de la serrure d'injection est le suivant. Après retrait des capot C2 et capuchon C1, l'autoinjecteur est prêt à être utilisé. Cette position initiale est représentée sur les figures 2a et 2b, ainsi que sur les figures 7a à 7c. En début d'actionnement, le manchon actionneur 10 coulisse vers l'intérieur du corps, et une extrémité supérieure 19 du manchon actionneur 10 va venir en contact avec un épaulement 49 du manchon de commande 4, comme illustré sur la figure 3a. Au moment où ce contact entre le manchon actionneur 10 et le manchon de commande 4 se produit, il demeure un petit espace entre le bord inférieur de la bague de blocage 23 et le manchon de commande 4, comme visible sur la figure 8c. Une poursuite du coulissement axial du manchon actionneur 10 va alors entrainer le manchon de commande 4 axialement vers le haut, ce qui va provoquer le contact entre le manchon de commande 4 et la bague de blocage 23, comme visible sur la figure 9c. Dans cette position, la serrure d'injection est toujours en position de blocage. Une poursuite du déplacement axial du manchon actionneur 10 va alors déplacer axialement vers le haut ladite bague de blocage 23, via ledit manchon de commande 4. Ce déplacement de la bague de blocage va libérer les billes 7 pour déclencher la serrure d'injection. Comme visible sur les figures 4a, 4b et 10a à 10c, dès que le bord inférieur de la bague de blocage 23 ne contacte plus les billes 7, celles-ci peuvent s'échapper radialement vers l'extérieur, libérant ainsi la tige de piston 5. Dans cette position, la serrure d'injection ne bloque plus la tige de piston 5, et l'injection commence sous l'effet du ressort d'injection 8. Comme visible sur la figure 11b, les billes 7 se positionnent dans une fenêtre du manchon de commande 4. Lorsque le piston P arrive vers la fin de la course d'injection, comme représenté sur les figures 5a et 5b, la tige de piston 5 va coopérer avec la tige 82 de la clé 80 pour tirer celle-ci axialement vers la bas. De ce fait, la tête 81 de la clé se déplace aussi axialement vers le bas, comme représenté sur les figures 12a à 12c, ce qui va libérer l'élément indicateur 70. Lorsque la tête 81 de la clé 80 ne bloque plus l'élément indicateur 70, comme visible sur les figures 13a à 13c, celui-ci est déplacé axialement vers le haut sous l'effet du ressort d'injection 8 qui s'appuie sur l'organe support 6, lui-même solidaire dudit élément indicateur 70. Ceci provoque une indication sonore et/ou tactile par contact soudain entre l'élément indicateur 70 et le corps 1, et une indication visuelle par les pattes 71 qui deviennent visibles dans les fenêtres 9 du corps 1. Lorsque la phase d'injection est terminée, le ressort 3 sollicite le manchon actionneur 10 vers sa seconde position projetée pour recouvrir l'aiguille A, comme visible sur les figures 6a et 6b.

Les figures 15a à 15g illustrent l'assemblage de la serrure d'injection.

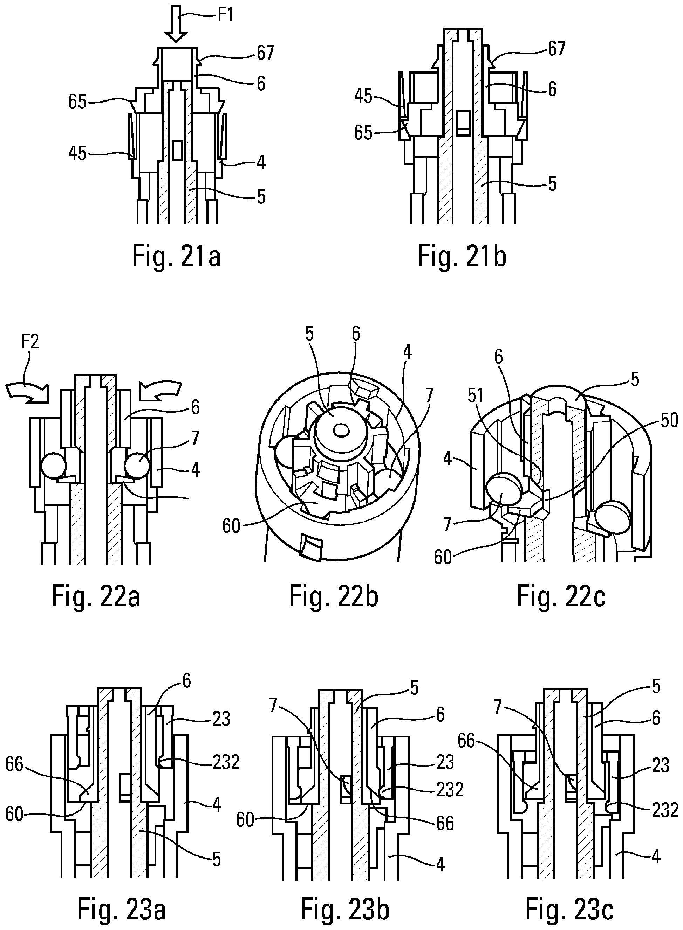

La tige de piston 5 est disposée dans le manchon de commande 4, puis l'organe support 6 est disposé dans ledit manchon de commande 4 autour de ladite tige de piston 5. Les figures 7a, 21a et 21b montrent l'encliquetage de premières projections radiales 65 de l'organe support 6 sous un épaulement 45 du manchon de commande 4 lorsque l'organe de support 6 est déplacé axialement vers le bas dans le sens de la flèche F1.

Ensuite, les billes 7 sont mise en place par en haut, selon les flèches F2 de la figure 22a, venant se positionner à l'intérieur du manchon de commande 4, sur l'anneau 60 de l'organe support 6.

Puis la bague de blocage 23 est assemblée dans ledit manchon de commande 4, autour dudit organe support 6. La bague de blocage 23 comporte avantageusement une partie inférieure chanfreinée 231, qui lors de l'assemblage de la bague de blocage 23 va pousser les billes radialement vers l'intérieur, comme illustré sur la figure 15e. De cette manière, les billes viennent en position de blocage, dans l'évidement 50 de la tige de piston 5, en contact contre ladite paroi inclinée 51 de la tige de piston 5. Comme visible sur les figures 7c, 23a à 23c, la bague de blocage 23 comporte des ergots 232 saillant radialement vers l'intérieur qui, lors de l'assemblage, viennent s'engager, notamment par encliquetage, sous une rampe inclinée 66 de l'organe support 6. Lors du déclenchement de la serrure d'injection, la bague de blocage 23 se désengage de l'organe support 6 pour libérer les billes 7. Ces ergots 232, de préférence de forme arrondie, renforcent la résistance de la serrure aux "drops tests", en empêchant un déplacement non souhaité de ladite bague de blocage 23 hors de sa position de blocage. Un autre avantage de la bague de blocage 23 est de créer un point de résistance lors de l'actionnement, notamment via l'encliquetage des ergots 232. Ce "point dur", qui doit être surmonté lors de l'actionnement, permet d'assurer que le patient arrive en fin de piquage pour injecter à la bonne profondeur. Ce "point dur" est positionné de plus de façon à permettre une course morte initiale du manchon actionneur 10, jusqu'à ce que le manchon de commande 4 vienne en contact avec la bague de blocage 23, permettant ainsi à l'utilisateur de faire des essais de positionnement de l'autoinjecteur avant de déclencher l'injection.

Dans les positions des figures 15f et 23c, la serrure d'injection est en position de blocage.

Ensuite, l'élément indicateur 70 vient s'assembler sur l'organe support 6, via des fenêtres 77 coopérant avec des secondes projections radiales 67 de l'organe support 6, visibles notamment sur la figure 7a.

La présente invention s'applique à des dispositifs utilisés notamment pour les traitements des maladies auto-immunes, par exemple du type arthrites rhumatoïdes, scléroses multiples, maladie de Crohn, pour les traitements contre le cancer, pour les traitements antiviraux, par exemple du type hépatites, pour les traitements contre le diabète, pour les traitements contre l'anémie ou pour les traitements de crises, par exemple en cas de choc anaphylactique.

Bien que la présente invention ait été décrite en référence à un mode de réalisation avantageux, il est entendu que diverses modifications sont possibles pour l'homme du métier sans sortir du cadre de la présente invention tel que défini par les revendications annexées.

## Revendications

1. Autoinjecteur comportant un corps (1) recevant un réservoir (S), ledit réservoir (S) contenant du produit fluide et comportant un piston (P) et une aiguille (A), tel qu'une seringue pré-remplie, ledit autoinjecteur comportant une tige de piston (5) adaptée à coopérer avec le piston (P) dudit réservoir (S), ladite tige de piston (5) étant déplaçable entre une position chargée et une position d'injection dans laquelle ladite tige de piston (5) a déplacé le piston (P) du réservoir (S) pour injecter le produit fluide à travers l'aiguille (A), un ressort d'injection (8) étant prévu pour solliciter ladite tige de piston (5) vers sa position d'injection, l'autoinjecteur comportant une serrure d'injection qui bloque ladite tige de piston (5) dans sa position chargée, ladite serrure d'injection comportant un manchon de commande (4), ledit manchon de commande (4) contenant ladite tige de piston (5) et ledit ressort d'injection (8), ladite tige de piston (5) comportant un évidement radial (50) recevant au moins un élément de blocage sensiblement sphérique (7), tel qu'une bille, mobile entre une position de blocage et une position de déblocage, ledit au moins un élément de blocage (7) étant sollicité radialement vers l'extérieur par ladite tige de piston (5) et étant retenu en position de blocage par une bague de blocage (23), ladite bague de blocage (23) étant déplaçable par rapport à ladite tige de piston (5) pour libérer ledit au moins un élément de blocage (7) et ainsi débloquer ladite serrure d'injection, permettant audit ressort d'injection de déplacer ladite tige de piston (5) vers sa position d'injection, **caractérisé en ce qu'**un organe support (6) est fixé, notamment encliqueté, dans ledit manchon de commande (4), ledit organe support (6) comportant un anneau (60) dont un côté axial est en contact avec ledit ressort d'injection (8) et l'autre côté axial supporte ledit au moins un élément de blocage sensiblement sphérique (7), ladite bague de blocage (23) étant engagée, notamment encliquetée, sur ledit organe support (6), ladite bague de blocage (23) étant désengagée dudit organe support (6) par ledit manchon de commande (4) lorsque ledit manchon de commande (4) est déplacé axialement lors de l'actionnement de l'autoinjecteur, pour débloquer ladite serrure d'injection.

2. Autoinjecteur selon la revendication 1, dans lequel ladite bague de blocage (23) comporte des ergots (232), de préférence de forme arrondie, qui sont, en position de blocage de la serrure d'injection, encliquetés sous une rampe inclinée (66) dudit organe support (6).

3. Autoinjecteur selon la revendication 1 ou 2, dans lequel ladite bague de blocage (23) comporte un bord inférieur chanfreiné (231) pour solliciter ledit au moins un élément de blocage radialement vers l'intérieur lors de l'assemblage de ladite bague de blocage (23) sur ledit organe support (6).

4. Autoinjecteur selon l'une quelconque des revendications précédentes, dans lequel ledit organe support (6) comporte des premières projections radiales (65) coopérant avec un épaulement (45) dudit manchon de commande (4) pour fixer ledit organe support (6) sur ledit manchon de commande (4).

5. Autoinjecteur selon l'une quelconque des revendications précédentes, dans lequel un élément indicateur (70) est fixé, notamment encliqueté, sur ledit organe support (6), ledit élément indicateur (70) coopérant après injection avec le corps (1) pour fournir à l'utilisateur une indication sonore et/ou tactile et/ou visuelle.

6. Autoinjecteur selon la revendication 5, dans lequel ledit organe support (6) comporte des secondes projections radiales (67) coopérant avec des fenêtres (77) dudit élément indicateur (70) pour fixer ledit élément indicateur (70) sur ledit organe support (6).

7. Autoinjecteur selon l'une quelconque des revendications précédentes, dans lequel ledit autoinjecteur comporte un manchon actionneur (10) comportant une extrémité de contact (11) destinée à venir en contact avec le corps de l'utilisateur, ledit manchon actionneur (10) s'étendant au moins partiellement à l'intérieur dudit corps (1) et étant déplaçable par rapport audit corps (1) entre des positions projetées, dans lesquelles ledit manchon actionneur (10) fait au moins partiellement saillie hors dudit corps (1), et une position d'actionnement, dans laquelle ledit manchon actionneur (10) est axialement déplacé vers l'intérieur dudit corps (1), ledit manchon actionneur (10) étant dans une première position projetée avant actionnement de l'autoinjecteur et dans une seconde position projetée après actionnement de l'autoinjecteur.

8. Autoinjecteur selon la revendication 7, dans lequel ledit manchon actionneur (10) comporte un bord supérieur (19) qui vient en contact dudit manchon de commande (4) lors de l'actionnement, de sorte que ledit manchon de commande (4) est déplacé axialement par ledit manchon actionneur (10) pour débloquer ladite serrure d'injection.

## Patentansprüche

1. Autoinjektor, aufweisend einen Körper (1), der einen Behälter (S) aufnimmt, wobei der Behälter (S) ein fluides Produkt enthält und einen Kolben (P) und eine Nadel (A), wie eine vorgefüllte Spritze, enthält, wobei der Autoinjektor eine Kolbenstange (5) aufweist, die dafür geeignet ist, mit dem Kolben (P) des Behälters (S) zusammenzuwirken, wobei die Kolbenstange (5) zwischen einer belasteten Position und einer Injektionsposition verschiebbar ist, in der die Kolbenstange (5) den Kolben (P) des Behälters (S) verschoben hat, um das fluide Produkt über die Nadel (A) zu injizieren, wobei eine Injektionsfeder (8) vorgesehen ist, um die Kolbenstange (5) in ihre Injektionsposition vorzuspannen, wobei der Autoinjektor einen Injektionsverschluss aufweist, der die Kolbenstange (5) in ihrer belasteten Position sperrt, wobei der Injektionsverschluss eine Steuermuffe (4) aufweist, wobei die Steuermuffe (4) die Kolbenstange (5) und die Injektionsfeder (8) enthält, wobei die Kolbenstange (5) eine radiale Vertiefung (50) aufweist, die mindestens ein in etwa kugelförmiges Sperrelement (7), wie eine Kugel, aufnimmt, die zwischen einer Sperrposition und einer Entsperrposition beweglich ist, wobei das mindestens eine Sperrelement (7) durch die Kolbenstange (5) radial nach außen vorgespannt ist und von einem Sperrring (23) in der Sperrposition gehalten wird, wobei der Sperrring (23) in Bezug auf die Kolbenstange (5) verschiebbar ist, um das mindestens eine Sperrelement (7) freizusetzen und somit den Injektionsverschluss zu entsperren, was es der Injektionsfeder ermöglicht, die Kolbenstange (5) in ihre Injektionsposition zu verschieben, **dadurch gekennzeichnet, dass** ein Trägerorgan (6) in der Steuermuffe (4) befestigt, insbesondere eingerastet ist, wobei das Trägerorgan (6) einen Ring (60) trägt, dessen eine axiale Seite mit der Injektionsfeder (8) in Kontakt steht und dessen andere axiale Seite das mindestens eine, in etwa kugelförmige Sperrelement (7) trägt, wobei der Sperrring (23) auf dem Trägerorgan (6) befestigt, insbesondere eingerastet ist, wobei der Sperrring (23) durch die Steuermuffe (4) von dem Trägerorgan (6) gelöst wird, wenn die Steuermuffe (4) bei Betätigung des Autoinjektors axial verschoben wird, um den Injektionsverschluss zu entsperren.

2. Autoinjektor nach Anspruch 1, wobei der Sperrring (23) vorzugsweise abgerundete Nocken (232) aufweist, die in der Sperrposition des Injektionsverschlusses auf einer geneigten Rampe (66) des Trägerorgans (6) eingerastet sind.

3. Autoinjektor nach Anspruch 1 oder 2, wobei der Sperrring (23) einen unteren angefasten Rand (231) aufweist, um das mindestens eine Sperrelement bei Montage des Sperrrings (23) auf dem Trägerorgan (6) radial nach innen vorzuspannen.

4. Autoinjektor nach einem der vorhergehenden Ansprüche, wobei das Trägerorgan (6) erste radiale Vorsprünge (65) aufweist, die mit einem Absatz (45) der Steuermuffe (4) zusammenwirken, um das Trägerorgan (6) auf der Steuermuffe (4) zu befestigen.

5. Autoinjektor nach einem der vorhergehenden Ansprüche, wobei ein Anzeigeelement (70) auf dem Trägerorgan (6) befestigt, insbesondere eingerastet ist, wobei das Anzeigeelement (70) nach der Injektion mit dem Körper (1) zusammenwirkt, um dem Benutzer eine akustische und/oder taktile und/oder visuelle Anzeige zu liefern.

6. Autoinjektor nach Anspruch 5, wobei das Trägerorgan (6) zweite radiale Vorsprünge (67) aufweist, die mit Fenstern (77) des Anzeigeelements (70) zusammenwirken, um das Anzeigeelement (70) auf dem Trägerorgan (6) zu befestigen.

7. Autoinjektor nach einem der vorhergehenden Ansprüche, wobei der Autoinjektor eine Betätigungsmuffe (10) aufweist, die ein Kontaktende (11) aufweist, das dazu gedacht ist, mit dem Körper des Benutzers in Kontakt zu geraten, wobei sich die Betätigungsmuffe (10) zumindest teilweise ins Innere des Körpers (1) erstreckt und in Bezug auf den Körper (1) zwischen vorstehenden Positionen, in denen die Betätigungsmuffe (10) zumindest teilweise aus dem Körper (1) vorsteht, und einer Betätigungsposition verschiebbar ist, in der die Betätigungsmuffe (10) zum Inneren des Körpers (1) axial verschoben ist, wobei sich die Betätigungsmuffe (10) vor der Betätigung des Autoinjektors in einer ersten vorstehenden Position und nach der Betätigung des Autoinjektors in einer zweiten vorstehenden Position befindet.

8. Autoinjektor nach Anspruch 7, wobei die Betätigungsmuffe (10) einen oberen Rand (19) aufweist, der bei Betätigung mit der Steuermuffe (4) in Kontakt gerät, so dass die Steuermuffe (4) durch die Betätigungsmuffe (10) axial verschoben wird, um den Injektionsverschluss zu entsperren.

## Claims

1. An autoinjector comprising a body (1) that receives a reservoir (S), said reservoir (S) containing fluid product and including a piston (P) and a needle (A), such as a pre-filled syringe, said autoinjector comprising a piston rod (5) that is adapted to co-operate with the piston (P) of said reservoir (S), said piston rod (5) being movable between a primed position and an injection position in which said piston rod (5) has moved the piston (P) of the reservoir (S) so as to inject the fluid product through the needle (A), an injection spring (8) being provided for urging said piston rod (5) towards its injection position, the autoinjector comprising an injection lock that blocks said piston rod (5) in its primed position, said injection lock including a control sleeve (4), said control sleeve (4) containing said piston rod (5) and said injection spring (8), said piston rod (5) including a radial recess (50) that receives at least one substantially-spherical blocking element (7), such as a ball, that is movable between a blocking position and an unblocking position, said at least one blocking element (7) being urged radially outwards by said piston rod (5) and being held in its blocking position by a blocking ring (23), said blocking ring (23) being movable relative to said piston rod (5) so as to release said at least one blocking element (7) and thus unblock said injection lock, enabling said injection spring to move said piston rod (5) towards its injection position, the autoinjector being **characterized in that** a support member (6) is fastened, in particular snap-fastened, in said control sleeve (4), said support member (6) including a ring (60) having an axial end that is in contact with said injection spring (8) and the other axial end supports said at least one substantially-spherical blocking element (7), said blocking ring (23) being engaged, in particular snap-fastened, on said support member (6), said blocking ring (23) being disengaged from said support member (6) by said control sleeve (4) when said control sleeve (4) is moved axially while the autoinjector is being actuated, so as to unblock said injection lock.

2. An autoinjector according to claim 1, wherein said blocking ring (23) includes lugs (232), preferably of rounded shape, that, in the blocking position of the injection lock, are snap-fastened below a sloping ramp (66) of said support member (6).

3. An autoinjector according to claim 1 or claim 2, wherein said blocking ring (23) includes a beveled bottom edge (231) for urging said at least one blocking element radially inwards while said blocking ring (23) is being assembled on said support member (6).

4. An autoinjector according to any preceding claim, wherein said support member (6) includes first radial projections (65) that co-operate with a shoulder (45) of said control sleeve (4) for fastening said support member (6) on said control sleeve (4).

5. An autoinjector according to any preceding claim, wherein an indicator element (70) is fastened, in particular snap-fastened, on said support member (6), said indicator element (70) co-operating with the body (1), after injection, so as to provide the user with an audible and/or tactile and/or visual indication.

6. An autoinjector according to claim 5, wherein said support member (6) includes second radial projections (67) that co-operate with slots (77) of said indicator element (70) for fastening said indicator element (70) on said support member (6).

7. An autoinjector according to any preceding claim, wherein said autoinjector includes an actuator sleeve (10) that includes a contact end (11) for coming into contact with the user's body, said actuator sleeve (10) extending inside said body (1) at least in part, and being movable relative to said body (1) between projecting positions in which said actuator sleeve (10) projects out from said body (1) at least in part, and an actuated position in which said actuator sleeve (10) is moved axially into said body (1), said actuator sleeve (10) being in a first projecting position before actuation of the autoinjector, and in a second projecting position after actuation of the autoinjector.

8. An autoinjector according to claim 7, wherein said actuator sleeve (10) includes a top edge (19) that, during actuation, comes into contact with said control sleeve (4), such that said control sleeve (4) is moved axially by said actuator sleeve (10) so as to unblock said injection lock.
